# EUROPÄISCHE PATENTSCHRIFT

(11) **EP 1 792 705 B1**
(45) Veröffentlichungstag und Bekanntmachung des Hinweises auf die Patenterteilung: **16.09.2009**
(21) Anmeldenummer: 07102739.5
(22) Anmeldetag: 05.07.2002
(51) Int. Cl.: B29C 65/00

(54) **Medizinische Kapseln für die Inhalation**
Medical inhalation capsules
Capsules médicales destinées à l'inhalation

(30) Priorität: 28.07.2001 DE 10137054
(43) Veröffentlichungstag der Anmeldung: 06.06.2007
(62) Teilanmeldung aus: 02754835.3
(73) Patentinhaber: Boehringer Ingelheim Pharma GmbH & Co. KG, 55216 Ingelheim am Rhein (DE)
(72) Erfinder: Hochrainer, Dieter, 57392 Schmallenberg (DE); Wachtel, Herbert, 55218 Ingelheim am Rhein (DE)
(74) Vertreter: Hammann, Heinz

(56) Entgegenhaltungen:
- EP-A- 0 290 638
- EP-A- 0 360 765
- EP-A2- 0 839 634
- WO-A-00/07572
- DE-A1- 2 352 707
- DE-A1- 19 629 223
- DE-B- 1 217 024
- FR-A1- 2 812 372
- GB-A- 1 011 727
- GB-A- 2 149 377
- JP-A- 2001 191 412
- US-A- 3 438 826
- US-A- 3 466 842
- US-A- 4 550 238

## Beschreibung

Die vorliegende Erfindung betrifft Inhalationskapseln, wie sie aus der WO-A-00 07 572 bekannt sind.Die Kapseln sind Einweg-Kapseln und enthalten bevorzugt eine Einzeldosis einer inhalativ applizierbaren pharmazeutischen Formulierung in Form eines Pulvers oder einer Flüssigkeit und sind von der Form und Funktion geeignet zur Verwendung in Pulverinhalatoren oder Flüssigkeitsverneblern zur Erzeugung von Aerosolen. Solche erzeugten Aerosole können beispielsweise inhaliert werden, so daß eine pharmazeutische Formulierung pulmonal appliziert wird.

### Stand der Technik

Kapseln mit pharmazeutischen Zubereitungen werden vielfältig in der Therapie und Diagnose von Krankheiten eingesetzt. Die Kapseln können oral verabreicht werden oder kommen in bestimmten medizinischen Vorrichtungen, wie Pulverinhalatoren, zum Einsatz. In der Regel bestehen die Kapseln aus zwei Teilen, einem Kapselkörper (Körper) und einer Kapselkappe (Kappe), die teleskopartig ineinander geschoben werden. Aber auch mehrteilige Kapseln sind bekannt. Die Kapseln bestehen meistens aus Gelatine, insbesondere Hartgelatine. Für einige spezielle Anwendungen bestehen die Kapseln zuweilen auch aus für den Menschen gut verdaulichen, wasserlöslichen Kunststoffen, um bei oraler Verabreichung den Wirkstoff in bestimmten Kompartimenten des Magen-Darm-Trakts freizusetzen. Im folgenden sind Beispiele für verschiedene Kapselmaterialien aufgeführt.

EP 0460921 beschreibt Kapseln aus Chitosan und Stärke, Getreidepulver, Oligosacchariden, Methacrylsäure-Methylacrylat, Methacrylsäure-Ethylacrylat, Hydroxypropylmethyl-Celluloseacetat, -succinat oder -phthalat.

Das Kapselmaterial zeichnet sich dadurch aus, daß der Inhalt erst im Dickdarm freigesetzt wird.

GB 938828 offenbart Kapseln für radioaktive Substanzen im therapeutischen oder diagnostischen Einsatz. Die Kapseln bestehen aus wasserlöslicher Gelatine, Methylcellulose, Polyvinylalkohol oder wasserlöslichen nichttoxischen Thermoplasten.

Die EP0312760 beschreibt ein Verfahren zum Versiegeln von Hartgelatine oder Stärkekapseln mit einem bestimmten Siegelmittel. Die Naht der Kapseln kann dabei von der Mittelebene der Längsachse der Kapsel verschoben sein.

Die DE 3430764 offenbart ein anderes Verfahren zum Verschließen von Hartgelatinekapseln. Bei dem Verfahren werden die Kapseln zunächst gefüllt und die beiden Kapselhälften teleskopartig ineinander gesteckt. Anschließend wird durch Anheben der Kapselkappe gegenüber dem Kapselkörper am Kapselkörper eine Kontaktzone freigelegt, wobei die Kapsel nicht geöffnet werden darf. In einem nächsten Schritt wird dann die Kontaktzone "klebrig" gemacht um anschließend wird die Kapselkappe wieder zurück auf ihren ursprünglichen Position geschoben und dabei mit der Kontaktzone in Berührung gebracht. Dieses Verfahren erfordert eine hohe Präzession bei der Ausführung, insbesondere da vermieden werden muss, die Kapsel beim Zurückführen der Kapselkappe auf den durch Erwärmung klebrig gemachte und damit gegenüber Verformung anfälligen Kapselkörper zu verformen. Auf Seite 32 der Anmeldung wird davon gesprochen, dass zum Halten und Führen der Kapselteile Werkzeuge notwendig sind, die keinerlei Toleranzen oder Spiel aufweisen.

Die US 4991377 offenbart ein weiteres Verfahren zum Verschweißen von Keratin- oder Gelatinekapseln. Bei dem Verfahren wird die geschlossene, Kapsel, welche aus zwei teleskopartig verbunden Teilen besteht, an der Nahtstelle mit Heißluft behandelt. Bei dem Verfahren sitzt der untere Bereich der Kapsel auf einem Halter. Die Patentschrift gibt weder einen Hinweis darauf wie eine Verformung der durch den Wärmestrahl erweichten Kapseln vermieden werden kann noch wie ein Erwärmen oder Verkohlen andere Kapselbereiche außerhalb der Nahtzone vermieden werden kann. Es wird auch nicht offenbart, wie die Inhaltsstoffe in der Kapsel vor der durch das Verschweißen bedingten Hitze in seine Qualität beeinträchtigt wird.

Die WO 00/07572 offenbart Kapseln für Inhalatoren , welche aus nicht-verdaulichem Kunststoff bestehen. Die dort beschriebenen Kapseln werden analog den handelsüblichen Hartgelatinekapseln verschlossen, das heißt die Kapselkappe wird teleskopartig auf den Kapselkörper aufgesteckt. Gegebenenfalls kann der dabei zwangsläufig entstehende Überlappungsbereich zwischen Kapselkappe und Kapselkörper verschweißt, verklebt oder banderoliert werden, um die Wasserdampfpermeabilität zu reduzieren. Alternativ kann die gesamte Kappe mit einem durchgehenden Schutzfilm überzogen werden oder der Spalt der Nahtstelle wird mit einem Füllstoff verschlossen. Einzelheiten zu den Verfahren zum Verschließen der Kappe werden nicht offenbart, insbesondere keine Schweißverfahren.

Die EP-A-0 360 765 offenbart Arznei enthaltende Kapseln mit einer teleskopartig auf einem Kapselkörper aufgesteckten Kapselkappe, wobei der Überlappungsbereich durch einen drehenden Heißgasstrahl verschweißt wird.

Zum Verschweißen von Kunststoffmaterialien sind aus dem Stand der Technik verschiedene thermische Verschweißungsarten bekannt. Dazu zählen Ultraschallverschweißen, Heißplatten- und Heiß-Werkzeug-Verschweißen, Heißgasverschweißen, Drehschweißen, Hoch-Frequenz-Spannungs-Verschweißen oder Induktionsschweißen.

Nicht bekannt dagegen sind derartige Verfahren zum Verschließen von Kunststoffkapseln für medizinische Inhalatoren, die bezüglich der Verwendung bestimmten Rahmenbedingungen unterworfen sind.

Es hat sich nun gezeigt, daß sich diese Schweißverfahren nicht ohne weiteres auf das Verschweißen der in der WO 00/07572 beschriebenen Kapselhälften übertragen lassen.

Zu den oben genannten Rahmenbedingungen, die einem einfachen Übertragen der aus dem Stand der Technik bekannten Verfahren auf Inhalationskapseln entgegenstehen, zählt beispielsweise, daß die Kapseln mit einer pharmazeutischen Formulierung gefüllt sind, deren pharmazeutische Qualität während des Verschweißens nicht vermindert werden darf.

Eine weitere Rahmenbedingung stellt die Abmessung und Wandstärke der zu verschweißenden Kapseln dar, insbesondere die dünne Wandung einer solchen Kapsel. Diese ist notwendig, da die Kapsel analog den bisher gebräuchlichen Hartgelatinekapseln in einem handelsüblichen Inhalator verwendet werden können soll. Dort muß sie nämlich in einfacher Art und Weise geöffnet werden. Werden die herkömmlichen Verfahren auf eine solche Kapsel angewandt, kommt es beispielsweise schnell zum Einbrennen von Löchern während des Verschweißens, besonders in den Bereichen der Kapsel, die außerhalb des Bereichs liegen, der verschweißt werden soll. Dieser Bereich, an dem die Nahtstelle ausbildet werden soll befindet sich naturgemäß ein einem Bereich, in dem die Wandungen der beiden miteinander zu verbindenden Teile überlappen. Daneben gibt es Bereiche, bei denen keine derartige Überlappung vorliegt. In diesen Bereichen kann die Kapsel durch den Schweißvorgang leichter beschädigt werden. Daneben besteht auch die Gefahr, daß bei zu dicken Kapselwandungen eine Schweißnaht an der Nahtstelle nicht durchgehend ausgebildet wird.

### Beschreibung der Erfindung

Mit der vorliegenden Erfindung sollen dichte Kunststoffkapseln für medizinische Inhalationsgeräte geschaffen werden, die eine pharmazeutische Formulierung enthalten. Bevorzugt enthalten diese Kapseln eine Einmaldosis der Formulierung. Die Kapseln werden im Kontext der vorliegenden Erfindung auch Einzeldosis-Kapseln genannt.

Ein für die vorliegenden Zwecke bevorzugter Inhalator wird beispielsweise in der WO 94/28958 beschriebenen (HandiHaler®), auf den hiermit in vollem Umfang Bezug genommen wird.

Die vorliegende Erfindung löst das oben beschriebene Problem, indem die Kapseln durch ein neues Schweißverfahren hergestellt werden, bei dem ausschließlich der zu verschweißende Bereich einer Inhalationskapsel mittels eines Energiestrahls in Form eines Heißgasstrahls oder eines Laserstrahls verschweißt wird. Dabei kommt es bevorzugt nur an der Nahtstelle zu einer Erwärmung des Kapselmaterials.

Damit besteht eine Aufgabe der Erfindung darin, daß die Bereiche einer Kunststoffkapsel, die nicht verschweißt werden sollen, nicht bis zum Schmelzbereich des Kunststoffs erwärmt werden.

Eine weitere Aufgabe besteht darin, daß die einzelnen Teile einer solchen Kunststoffkapsel für Inhalatoren fest zusammengehalten werden.

Eine weitere Aufgabe besteht darin, die einzelnen Teile einer Kunststoffkapsel so miteinander zu verbinden, dass sie ohne Beschädigung der Kapsel nicht mehr getrennt werden können. Dies erleichtert dem Verwender festzustellen, dass die Kapsel zuvor nicht bereits geöffnet wurde.

Eine weitere Aufgabe besteht darin, Kunststoffkapseln zu schaffen, bei denen es nicht durch den in der Kapsel durch das Verschweißen erzeugten Überdruck zu einer Beschädigung der Kapsel kommt bzw. in denen ein solcher Überdruck minimiert wird.

Eine weitere Aufgabe besteht darin, Kunststoffkapseln zu schaffen, bei denen die pharmazeutische Qualität der Arzneimittelformulierung in der Kapsel, die erfindungsgemäß eine Inhalationsformulierung ist, während des Verschweißens nicht gefährdet ist.

Eine weitere Aufgabe der Erfindung besteht darin, die Nahtstelle von Inhalationskapseln der vorstehend beschriebenen Art in industriellem Maßstab unter GMP-Bedingungen (Good manufacturing practice) zu verschweißen.

Die zu verschweißenden Kapseln bestehen aus nicht-wasserlöslichen, bevorzugt hydrophoben Kunststoffen, die die pharmazeutische Qualität der Inhaltsstoffe selbst nicht wesentlich beeinflussen, aber die Einsatzfähigkeit der gefüllten Kapsel im Hinblick auf ihre Funktion, die Dauer der Verwendung und/oder die Klimazone verbessern und in verschiedenen Stufen von der Herstellung bis zur Verwendung von Vorteil sind.

Die im Anspruch definierte erfindungsgemäße Kapsel besteht aus mindestens zwei Teilen, einem Kapselkörper (Körper) und mindestens einer Kapselkappe (Kappe), die so miteinander verbunden werden können, daß ein stabiler, abgeschlossener Hohlraum von definiertem Volumen gebildet wird, der die pharmazeutische Formulierung beinhaltet. Die Dimension der Kapsel ist derart, daß sie in gängigen mit Kapseln bestückten Pulverinhalatoren eingesetzt werden können, wie sie z.B. in den Patentschriften DE 33 45 722 (Inhalator Ingelheim M), EP 0 591 136 (Inhalator Ingelheim) oder in der deutschen Offenlegungsschrift DE 43 18 455 ("HandiHaler®") beschrieben sind. Besonders bevorzugt ist die Kapselgröße 3.

In einer Ausführungsform ist der Kunststoff der Kapsel für den Menschen nicht verdaubar, so daß bei oraler Einnahme der Wirkstoff nicht freigesetzt wird. Das hat den Vorteil, daß ein versehentliches Schlucken der Kapsel zu keiner nachhaltigen Beeinträchtigung der Gesundheit führen kann. Insbesondere gilt dies für Kleinkinder oder ältere Menschen.

Bevorzugt werden Kunststoffe verwendet, die spritz- oder blasgußtechnisch verarbeitet werden können und/oder Kunststoffe für deren Verarbeitung zur Kapselkappe oder zum Kapselkörper kein Formtrennmittel notwendig ist, das ein Anhaften der Inhaltsstoffe an die Kapselwand bewirken kann. Das hat den Vorteil, daß das Innere der Kappe oder des Körpers nicht vom Formtrennmittel gereinigt werden muß, um z.B. den amtlichen Bestimmungen (z.B. nach DAB (Deutsches Arzneibuch)) genüge zu tun, die die Verwendung von Formtrennmitteln für Primärpackmittel einschränken. Bevorzugt sind Thermoplaste, die ein stoffschlüssiges Verschweißen der Kapselhälften miteinander ermöglichen.

Bevorzugte Materialien haben die Eigenschaft, daß möglichst wenig, bevorzugt kein Pulver an ihnen haften bleibt.

Zu den geeigneten Kunststoffen für das erfindungsgemäße Verfahren zählen Polyethylen LD (low density), Polyethylen HD (high densitiy), Polystyrol, Acrylnitril-Butadien-Styrol, Polypropylen, Polymethylmethacrylat, Polyvinylchlorid, Polyoxymethylen, Polycarbonat, Polyester oder Polyethylenterephthalat

In bevorzugten Ausführungen ist der Kunststoff Polyethylen, insbesondere Polyethylen mit einer Dichte zwischen 900 und 1000 kg/m³, bevorzugt von 960 kg/m³ (high-density Polyethylen).

In einer bevorzugten Ausführungsform besitzt der Kunststoff keine ausgeprägte Adhäsion für pharmazeutisch-chemische Stoffe, insbesondere für Partikel mit lungengängiger Größe, so daß bei Verwendung der Kapsel in einem der oben beschriebenen Inhalatoren der gesamte Inhalt der Kapsel freigesetzt werden kann. Das hat den Vorteil, daß eine exaktere Dosierung, insbesondere des lungengängigen Feinanteils möglich ist.

In einer weiteren Ausführungsform besteht die Kapsel aus einem Kunststoff mit einer Shorehärte D von 65 bis 73. Ein Kunststoff dieser Härte zerspringt nicht, wenn er durchstochen oder aufgeschnitten wird, ist aber gleichzeitig starr genug, daß sich das entstandene Loch nicht von selbst verschließt. Der Vorteil eines solchen Materials ist, daß während des Öffnens, Aufstechens oder Aufschneidens der Kapsel im Pulverinhalator keine Teile aus der Kapsel abgesprengt werden, die dann während des Inhalierens eingeatmet werden könnten.

In einer Ausführungsform ist die Kunststoffkapsel so stabil, daß sie entlang der Längsachse oder der Querachse einer Kraft bis zu 15 N standhält. Der Vorteil besteht darin, daß die Kapsel besser an die Beanspruchungen angepaßt ist, die bei der Herstellung, dem Füllen, Verpacken, Transportieren u.ä. auf die Kapsel einwirken.

In einer weiteren Ausführungsform besitzt das Material der Kapsel einen Permeationskoeffizienten für Wasserdampf von weniger als 10⁻¹³ kg/(m s Pa), bevorzugt weniger als 1,3 x 10⁻¹⁴ kg/(m s Pa). Bevorzugt liegt der Koeffizient zwischen 10⁻¹⁵ und 5 x 10⁻¹⁶ kg/(m s Pa), besonders bevorzugt zwischen 5 x 10⁻¹⁶ und 2 x 10⁻¹⁶ kg/(m s Pa). Der Vorteil dieser Eigenschaft besteht darin, daß die Inhaltsstoffe der Kapsel auch in geographischen Zonen mit hoher Luftfeuchtigkeit vor Wasser geschützt sind.

Die erfindungsgemäße Kapsel kann in allen Arten von Pulverinhalatoren verwendet werden, bei denen die zu inhalierende Zubereitung durch eine Kapsel eingebracht wird.
In einer bevorzugten Ausführungsform sind Kappe und Körper der Kapsel von gegenseitig ähnlicher, zylinderartiger Form, bestehend aus einem in sich geschlossenen Mantel mit jeweils einer geschlossenen und einer offenen Seite. Dabei sind Form und Größe der Kappe und der Kapsel dergestalt, daß der Körper mit seinem offenen Ende teleskopartig so in das offene Ende der Kappe hinein geschoben werden kann, daß die Kappe fest mit dem Körper verbunden ist.

In einer bevorzugten Ausführungsform haben die Kappe und der Körper die Form eines Zylinders mit rundem Querschnitt und konvexer, nahezu halbkugelförmiger geschlossener Unterseite und bestehen beide aus high-density Polyethylen mit einer Dichte zwischen 950 und 1000 kg/m³.

In einer speziellen Ausführungsform sind Kappe und Körper mit Verschlußeinrichtungen versehen, die beim vorläufigen und/oder endgültigen Verschließen der Kapsel von Vorteil sind.
In einer solchen Ausführungsform können sich auf dem Innenmantel der Kappe punktförmige Erhebungen und auf dem Außenmantel des Körper etwas größere punktförmige Vertiefungen befinden, die so angeordnet sind, daß beim Verschließen der Kapsel die Erhebungen in die Vertiefungen einrasten. Alternativ können die Erhebungen auf dem Außenmantel des Körpers und die Vertiefungen auf dem Innenmantel der Kappe ausgebildet sein. Bevorzugt sind Anordnungen, bei denen die Erhebungen oder Vertiefungen jeweils ringförmig oder spiralförmig um den Mantel angeordnet sind. Anstelle der punktförmigen Gestaltung der Erhebungen und Vertiefungen können diese auch durchgehend den Mantel der Kappe bzw. des Körpers ringförmig umlaufen. Leztere Rasten sind bevorzugt.
In einer Ausführungsform sind auf dem Innenmantel der Kappe und dem Außenmantel des Körpers ein oder mehrere ringförmig umlaufende Erhebungen derart ausgebildet, daß sich im geschlossenen Zustand der Kapsel eine Erhebung der Kappe jeweils neben einer Erhebung des Körpers befindet.
In den Ausführungsformen mit besagten ringförmigen Vertiefungen und/oder Erhebungen können diese durchgängig oder unterbrochen sein.
In einer weiteren Ausführungsform sind auf der Außenseite des Körpers nahe dem offenen Ende Erhebungen und in der Kappe nahe dem offenen Ende Löcher so ausgebildet, daß die Erhebungen des Körpers im geschlossenen Zustand der Kapsel in die Löcher der Kappe einrasten. Die Erhebungen können dabei derart sein, daß die Kappe jederzeit ohne Beschädigung der Kapsel geöffnet werden kann oder aber daß die Kapsel nach einmaligem Verschließen nicht mehr zerstörungsfrei geöffnet werden kann.

Bevorzugt sind Kapseln mit einem oder mehrerer solcher Einrastmechanismen (Rasten) (beipsielsweise zwei umlaufende Rillen).

Besonders bevorzugt sind Kapslen mit wenigstens zwei solcher Einrastmöglichkeiten, die die beiden Kapselteile unterschiedlich stark fixieren. In einem solchen Teil kann eine erste Einrastmöglichkeit nahe den Öffnungen der Kapselkappe und des Kapselkörpers ausgebildet sein und eine zweite etwas weiter zum geschlossenen Ende der Kapsleteile versetzte. Die erste Einrastmöglichkeit fixiert dabei die beiden Kapselteile weniger stark als die zweite.

Diese Variante hat den Vorteil, dass die Kapselkappe und der Kapselkörper vor dem Befüllen zunächst über den ersten Einrastmechanismus vorläufig miteinander verbunden werden können. Zum Befüllen der Kapsel werden dann die beiden Kapselteile wieder getrennt. Nach dem Befüllen werden die beiden Kapselteile soweit zusammengesteckt, bis der zweite Satz Rasten die Kapselteile fest fixiert.

In einer weiteren Ausführungsform ist auf der Außenseite des Körpers ein Wulst ausgebildet, der senkrecht zu der Verbindungsachse zwischen Kappe und Körper ringsum den Körper läuft. Der Wulst dient als Stopper für die Kapsel, wenn diese über den Körper gesteckt wird, um ein Durchstoßen der Kappe mit dem Körper zu verhindern. Der Bereich zwischen offenem Ende des Körpers und dem Wulst entspricht dem Bereich des Körpers, über den die Kappe geschoben werden kann. Der Wulst ist so auf dem Körper lokalisiert, daß die Kappe weit genug über den Körper geschoben werden kann, um einen festen Verschluß zwischen Kappe und Körper zu bewirken. D.h. der Wulst befindet sich z.B. nicht unmittelbar an der offenen Seite des Körpers. Die Seite des Wulstes, die zum offenen Ende des Körpers zeigt, steht als senkrechte Kante so auf der Außenwand des Körpers, daß die Kappe beim Verschließen nicht über den Wulst hinweg geschoben werden kann. Die Seite des Wulstes, die zum geschlossenen Ende des Körpers weist, kann in Form einer nahezu rechtwinkeligen Kante ausgebildet sein oder sich zum geschlossenen Ende des Körpers hin verflachen. Die Ausbildung einer nahezu rechtwinkeligen Kante kann bei einer losen Einpassung der Kapsel in einen Kapselhalter von Vorteil sein, die Variante mit sich verflachendem Wulst bei einer festen Einpassung. Der Wulst kann durchgängig oder unterbrochen sein.

In einer bevorzugten Ausführungsform verflacht sich der Wulst kontinuierlich zum geschlossenen Ende des Körpers und steht mit seiner zum offenen Ende des Körpers orientierten Seite senkrecht auf dem Kapselkörper auf. Dabei ist die Höhe der so gebildeten Kante derart, daß die Kante im geschlossenen Zustand der Kapsel nicht über die Kapselkappe hinaus ragt, so daß der Übergang von Kapselkappe zu Kapselkörper stufenlos ist.

Alternativ dazu kann anstelle des Wulstes der Durchmesser des Kapselkörpers sprunghaft an einer Stelle verkleinert sein, so daß der zur Öffnung orientierte Durchmesser kleiner (oder größer) als der zum geschlossenen Ende des Körpers hin orientierte Durchmesser ist. Dabei können Kappe und Körper so ausgebildet sein, daß die Kapsel im geschlossenen Zustand an der Nahtstelle stufenlos ist.

In einer anderen Alternative ist die Form der Wandung der Kappe im Öffnungsbereich genau umgekehrt ausgebildet wie die Form der Wandung der Öffnung der Kapsel. D.h. die Wandung der Kappe verbreitert sich im Bereich der Öffnung unter Ausbildung einer innenliegenden Kante. Auch in dieser Ausführungsform ist der Außenmantel bevorzugt plan. Bei dieser Ausführungsform wird die Kappe soweit auf die Kapsel gesteckt bis sich die Kante der Kappe und die Kante der Kapsel berühren.

In einer weiteren bevorzugten Ausführungsform befindet sich der Wulst auf der Innenseite des Kapselkörpers. Die Kante des Wulstes, auf der im geschlossenen Zustand der Kappe die Kapselkappe aufliegt, ist dann der Außenseite des Kapselkörpers zugewandt. In einem solchen Fall wird die Kapselkappe nicht auf den Kapselkörper aufgesteckt, sondern in den Kapselkörper hineingesteckt. Dabei können Kappe und Körper so ausgebildet sein, daß die Kapsel im geschlossenen Zustand an der Nahtstelle stufenlos ist. Im Rahmen der vorliegenden Erfindung werden die beiden Aufsteckmöglichkeiten der Kapselkappe über oder in den Kapselkörper als gleichwertig beschrieben, d.h. wird eine der beiden Varianten beschrieben, gilt analoges für die andere Variante.

In einer anderen Ausführungsform ist die Kante des Kapselkörpers als U-förmiger Umlauf ausgebildet, in den die Kante der Öffnung der Kapselkappe hineingesteckt wird.

In einer weiteren Ausführungsform weisen beide Öffnungen einen solchen U-fömigen Umlauf auf.

Wieder in einer anderen Ausführungsform ist die Kante der Öffnungen der Kappe und des Körpers verbreitert und zur Öffnung hin zeigend stufenlos ausgebildet.
Die Stärke der Wände der Kappe und des Körpers können über den Gesamtbereich variieren. So ist die Wandstärke in der Regel in den abgerundeten Bereichen der Kappe oder des Körpers oder an der Stelle des Körpers, an der der Wulst ausgebildet ist, größer als in den Bereichen, in denen die Wände geradlinig verlaufen. In einer Ausführungsform haben die Wände der Kappe und des Körpers eine Dicke von 0,1 mm bis 0,5 mm, bevorzugt weist die Kapsel eine mittlere Wandstärke von 0,1 mm bis 0,4 mm, besonders bevorzugt 0,2 mm bis 0,4 mm auf.

Der Kapselkörper weist im Bereich seiner Öffnung, insbesondere an seiner Kante eine Dicke von 0,15 mm bis 0,35 mm, bevorzugt 0,225 mm bis 0,275 mm, besonders bevorzugt 0,25 mm auf.

Die Kapselkappe weist im Bereich ihrer Öffnung, insbesondere an ihrer Kante eine Dicke von 0,25 mm bis 0,45 mm, bevorzugt 0,325 mm bis 0,375 mm, besonders bevorzugt 0,35 mm auf.

Die Länge der Kapsel beträgt 8 mm bis 30 mm, bevorzugt 13 bis 17mm, besonders bevorzugt 15,5 mm bis 16 mm. Der Durchmesser der Kapsel beträgt 4 mm bis 7 mm, bevorzugt 5,3 mm bis 6,3 mm. Besonders bevorzugt 5,75 bis 5,95 mm.
Eine bevorzugte Kapsel weist eine Länge von 15,9 mm, einen Durchmesser des Kapselkörpers von 5,57 mm und einen Durchmesser der Kapselkappe von 5,83 mm auf. Die bevorzugte Wandstärke des Kapselkörpers beträgt 0,25 mm und die der Kapselkappe 0,35 mm.

In einer möglichen Ausführungsform sind an der Außenseite der Kapsel Noppen ausgebildet, in einer anderen drei oder mehr Rippen, die parallel zur Längsachse der Kapsel verlaufen. Der Vorteil dieser Einrichtungen besteht darin, daß die Kapsel aus einer Kapselhalterung, wie sie z.B. in den oben genannten Pulverinhalatoren verwendet werden, so herausgenommen werden kann, daß sie nicht beschädigt wird oder aufgeht. Die Rippen oder Noppen können über die gesamte Außenseite der Kapsel hinweg verlaufen oder nur einen Teil davon bedecken. Alternativ können sie nur an der Kappe ausgebildet sein oder nur in dem Bereich des Körpers, der im geschlossenen Zustand nach außen sichtbar ist. Die Rippen verlaufen parallel zur Längsachse der Kapsel und bewirken, daß die Kapsel senkrecht in besagter Kapselhalterung fixiert ist. Im Fall eines kreisförmigen Querschnitts der Kapsel sind die Rippen bevorzugt so angeordnet, daß der Querschnitt der Kapsel keine Rotationssymmetrie um die Mittelachse aufweist. In einer solchen Ausführungsform können die Rippen nur in dem Bereich des Körpers ausgebildet sein, der im geschlossenen Zustand der Kapsel sichtbar ist. Eine solche Ausführungsform verhindert das Festklemmen der Kapsel in einem Kapselhalter.
In einer Ausführungsform ohne Wulst, aber mit Rippen an dem Teil des Körpers, der im geschlossenen Zustand der Kappe sichtbar ist, sind die Rippen so ausgebildet, daß die zum offenen Ende des Körpers orientierten Enden der Rippen die Aufgabe des Wulstes erfüllen, nämlich als Stopper für die Kappe zu dienen beim Zusammenfügen der Kappe mit dem Körper.

In einer weiteren Ausführung beschreiben die Mäntel der Kappe und der Körper einen hohlen Zylinder mit rundem, ovalem, drei-, vier-, sechs-, acht- oder mehreckigem Querschnitt, wobei die jeweilige Oberseite offen und die Unterseite geschlossen ist. Die geschlossene Unterseite kann flach oder konvex sein. Die eckigen Ausführungsformen haben z.B. den Vorteil, daß sie platzsparender gelagert werden können als die runden.

In einer Ausführungsform ist die Elongation der Kapsel (Abstand vom geschlossenen Ende des Körpers zum geschlossenen Ende der Kappe in Relation zum Durchmesser bei geschlossener Kapsel) größer 1, in einer Ausführungsform ist die Elongation gleich 1 und in wieder einer anderen Ausführungsform ist die Elongation kleiner 1. Letzteres hat den Vorteil, daß der Körper eine größere Öffnung zum Füllen aufweist.
Bei einer der Ausführungsformen mit einer Elongation gleich 1 sind Kappe und Körper dergestalt, daß die geschlossene Kapsel die Form einer Kugel hat, was für ein automatisches Beladen eines Pulverinhalators mit der Kapsel aus einem Reservoir von Vorteil sein kann.

Aus der Beschreibung wird ersichtlich, daß die erfindungsgemäße Kapsel besonders geeignet ist, jedwegliche pulverförmige und zur Inhalation geeignete pharmazeutische Formulierung aufzunehmen. In einer besonderen Verwendungsform enthält die Kapsel als Wirkstoff Cromoglicinsäure, Reproterol, Beclomethason, Terbutalin, Salbutamol, Salmeterol, Ketotifen, Orciprenalin Fluticason, Insulin, lpratropium, Tiotropium, Dexamethason, Bambuterol, Tiotropium, Budesonid, Fenoterol, Clenbuterol Prednisolon, Prednison, Prednyliden, Methylprednisolon, Formoterol, Nedocromil, deren pharmakologisch verträglichen Salze oder Gemische oder ein anderes für Inhalationszwecke geeignetes Kortisonpräparat oder Atropinderivat.

In einer bevorzugten Verwendungsform enthält die Kapsel Ipratropiumbromid oder Tiotropiumbromid.

Die verschweißten Kapseln können außer Pulverfüllungen auch Flüssigkeiten aufnehmen und sind dann auch für neuartige Flüssigkeits-Inhalatoren geeignet.

Zum stoffschlüssigen Verschließen der wenigstens zwei teleskopartig ineinander einsetzbaren Teilelemente der Kapsel eignet sich besonders ein Verfahren, bei dem ein örtlich eng begrenzter Energiestrahl aus Heißgas oder Laserlicht in einer Relativbewegung wenigstens einmal vollständig um die Senkrechtachse des überlappenden Bereichs der Kapselelemente herumgeführt wird, so daß das Kapselmaterial in diesem Bereich angeschmolzen, aber nicht zerstört wird und eine stoffschlüssige Nahtstelle auf diesem Bereich ausgebildet wird.

Die Rotationsbewegung kann entweder dadurch bewirkt werden, daß sich die Kapsel um ihre Achse, die senkrecht zur Ebene der Schweißnaht steht, im Energiestrahl dreht oder der Energiestrahl um die Kapsel herum geführt wird.

Die Kapselnaht wird dann innerhalb einer Umdrehung oder mehrerer Umdrehungen verschweißt.

Bevorzugt sind Schweißverfahren, bei denen die stoffschlüssige Schweißnaht innerhalb von mehreren Umdrehungen ausgebildet wird, da dadurch eine bessere Steuerung des Schweißvorgangs erreicht werden kann. Beispielsweise kann hierdurch das Einbrennen von Löchern in die Kapselwand verhindert werden.

Bevorzugt werden in dem Verfahren Heißgas und Laserlicht als Energiequellen eingesetzt. Bei Verwendung von Laserlicht wird im Kapselmaterial die für das Verschweißen notwendige thermische Energie durch Absorption induziert.

Der Verschweißvorgang selbst kann dabei entweder direkt an der Nahtstelle der beiden Kapselteile stattfinden oder im überlappenden Teil der beiden Kapselhälften.

Die dabei entstehende Schweißnaht wird als eine oder mehrere geschlossene Linie(n) entlang des Kapselumfangs parallel zu und zwischen den beiden Ebenen, die die Öffnungen der Kappe und des Körpers aufspannen, ausgebildet. Bevorzugt wird die Naht durch das Verfahren rundum dicht verschlossen.

Neben in sich geschlossenen Linien als Schweißnaht, sind auch Spirallinien möglich. Die Gestalt der Schweißnähte ist jedoch nicht auf geradlinige Formen beschränkt, sondern kann auch, gezackt meanderförmig oder in einer beliebigen anderen Form um den Kapselmantel umlaufend ausgeführt werden. Die Schweißnaht kann auch als Punktverschweißung durchgeführt werden.

Bevorzugt wird die Schweißnaht an einer Stelle ausgebildet, an der Kapselkörper und Kapselkappe gerade zu überlappen beginnen.
Je nach angewendetem Verfahren können auch mehrere Schweißnähte angebracht werden. Diese können beispielsweise parallel zu einander ausgebildet werden.

Bei mehrteiligen Kapseln sind an allen Verbindungsstellen Schweißnähte erforderlich, um die gewünschte Halte- und Dichtwirkung zu erzielen. Bei zwei- und mehrteiligen Kapseln erhöhen mehrere nebeneinanderliegende Schweißnähte die Dichtsicherheit.

In Abhängigkeit der Größe von Kapselkörper und Kapselkappe kann die Lage der Schweißnähte mittig zur Längsachse gewählt werden. Bevorzugt ist jedoch eine asymmetrische Lage, um möglichst großen Abstand zur Kapselfüllung zu gewähren.

Dies kann beispielsweise auch dadurch erreicht werden, daß entweder der Kapselkörper länger ist als die Kapselkappe oder *vice versa.* In diesem Zusammenhang wird unter dem Begriff Länge der Abstand zwischen der Öffnung eines jeden der beiden Kapselhälften und der gegenüberliegenden geschlossenen Seite verstanden. Bevorzugt befindet sich die Naht im oberen Drittel der geschlossenen Kapsel. Das heißt, daß der Anteil des Kapselkörpers vom geschlossenen Boden bis zur Stelle der Ausbildung des vorstehend beschriebenen Wulstes, der die Stelle markiert, bis wohin das offene Ende der Kapselkappe über die Öffnung des Kapselkörpers auf letzteren gesteckt wird, ca. 2/3 der Gesamtlänge der geschlossenen Kapsel Beträgt.

Eine solche Kapsel hat den Vorteil, daß die Füllhöhe der Formulierung innerhalb der Kapsel unterhalb der Nahtstelle liegen kann, so daß die Gefahr, die Qualität der Formulierung durch den Verschweißvorgang zu reduzieren, deutlich herabgesenkt wird.

Die Füllhöhe der Kapsel kann dabei entsprechend der Verschweißungstemperatur und der Temperaturempfindlichkeit der pharmazeutischen Formulierung angepasst werden.

Die vorliegende Erfindung betrifft daher auch eine solche Kunststoffkapsel mit asymmetrischer Lage der Schweißnaht. Die anderen Charakteristika der erfindungsgemäßen Kapsel wurden eingangs ausführlich beschrieben.

Die Verwendung einer Meßeinrichtung ermöglicht die Beurteilung der Schweißtemperatur und des Fließverhaltens des Kapselmaterials während des Schweißvorgangs und damit eine gezielte Steuerung.

Das Schweißverfahren findet bevorzugt unter Rückkopplung mit den Daten aus der Meßeinrichtung statt, um eine gleichbleibend hohe Qualität der Schweißnaht zu erreichen. Dadurch kann ein gleichmäßiger Energieeintrag an der Schweißstelle erhalten werden.

Die Rückkopplung gleicht Schwankungen z.B. der Materialstärke sowie von Umwelteinflüssen aus, indem entweder die Drehgeschwindigkeit der Relativrotation oder die zugeführte Energie nachgeregelt wird. In diesem Zusammenhang ist bevorzugt die Aufschmelzung eines möglichst kleinen Fleckes und einer Temperatur- bzw. Strahlungsmessung genau an dieser Stelle. Durch Drehung wird dann die Schweißnaht erzeugt.

In dem Verfahren werden die zugeführte Energie und die relative Rotationsgeschwindigkeit bevorzugt so aufeinander abgestimmt, daß das Kapselmaterial gerade zum Anschmelzen gebracht wird. Durch einmaliges oder mehrfaches Wiederholen dieses Vorgangs wird eine stoffschlüssige Schweißnaht erhalten.

Nachstehend werden Temperaturen genannt, die notwendig sind, um bevorzugte Kapselmaterialien zum Anschmelzen zu bringen:
Die Beispiele haben keinen limitierende Charakter auf die der Materialien.

| Material | Verarbeitungs-Temperatur |
|---|---|
| Polyethylen LD | 160°C - 260°C |
| Polyethylen HD | 260°C - 300°C |
| Polystyrol | 170°C - 280°C |
| Acrylnitril-Butadien-Styrol | 210°C - 275°C |
| Polypropylen | 250°C - 270°C |
| Polymethylmethacrylat | 210°C - 240°C |
| Polyvinylchlorid | 170°C - 210°C |
| Polyoxymethylen | 200°C - 210°C |

Der Schmelztemperatur entsprechend wird der Energiestrahl, seine Entfernung zur Kapseloberfläche und seine mittlere Verweildauer auf der Kapseloberfläche so eingestellt, daß das Kapselmaterial aufgeschmolzen wird, ohne daß ein Loch in die Kapselwand eingebrannt wird.

Die Rotationsgeschwindigkeit des Energiestrahls um die Kapsel, beziehungsweise der Kapsel im Energiestrahl, kann zwischen 0,01 Umdrehungen pro Sekunde und maximal 40 Umdrehungen pro Sekunde betragen. Im Fall des Laserschweißens kann sie bevorzugt 0,1 bis 20 Umdrehungen pro Sekunde, im Fall der Heißgasverschweißung bevorzugt 0,2 bis 2 Umdrehungen pro Sekunde betragen.

Damit ergibt sich eine Umfangsgeschwindigkeit der bevorzugten Kapsel mit einer Länge von 15,9 mm und einem Durchmesser der Kapselkappe von 5,83 mm und einem Durchmesser des Kapselkörpers 5,57 mm von ca. 0,18 mm pro Sekunde bis zu 732 mm pro Sekunde. Im Fall des Laserschweißvefahrens beträgt die Umfangsgeschwindigkeit bevorzugt 1,8 mm pro Sekunde bis 366 mm pro Sekunde und im Fall des Heißgasverschweißens 3,7 mm pro Sekunde bis 37 mm pro Sekunde.

Die Anzahl der Umdrehungen Kapsel/Energiestrahl kann für einen Verschweißungsvorgang bis zu 40 betragen, bevorzugt bis zu 20. Im Fall des Laserschweißverfahrens beträgt sie bevorzugt 2 bis 3, im Fall des Heißgasverschweißens 5 bis 8.

Bei dem Schweißverfahren werden die Kapseln und die Energiequelle bevorzugt mit einer bestimmten Vorschubgeschwindigkeit aneinander herangebracht. Bevorzugt sind Vorschubgeschwindigkeiten von 0,1 cm pro Sekunde bis 10 cm pro Sekunde, besonders bevorzugt 1 cm pro Sekunde bis 5 cm pro Sekunde.

Durch das Verfahren können die Kapseln mit einer Taktzeit von bevorzugt weniger als 10 Sekunden verschweißt werden. Stärker bevorzugt sind Verfahren, bei denen die Kapsel innerhalb von 5 Sekunden, noch stärker bevorzugt innerhalb von 1 Sekunde verschweißt wird.

Damit ergibt sich ein weiterer Vorteil des Verfahrens: Durch die kurze Schweißdauer wird eine Blasenbildung durch Aufheizen des in der Kapsel eingeschlossenen Gases vermieden.

Die durch das Verfahren geschaffenen Kapseln haben bevorzugt eine Symmetrieachse Cₙ (n=Zähligkeit der Symmetrie) und einer im Idealfall dazu senkrechten Symmetrieebene. Letztes Kriterium ist auf zylindrische Kapseln mit bezogen auf die Längsachse mittiger Schweißnaht beschränkt. Besonders bevorzugt sind rotationssymetrische Kapseln.

Dabei ist es ist nicht zwingend, daß der Umfang der Kapseln kreisförmig ist. Der Umfang kann auch vieleckig oder elliptisch sein.

Die äußerlich bevorzugte Gestalt der Kapsel weist keine Stufen auf. Besonders bevorzugt sind Kunststoffkapseln nach der Offenlegungsschrift DE 198 35 346 A1.

Im Fall der Laserverschweißung und Ausführungsformen der Kapseln mit vieleckigem oder elliptischem Querschnitt werden die Kapselnähte entweder unfokussiert in einem mittleren Arbeitsabstand mit einer mittleren Dosis bestrahlt oder bevorzugt mit aktiver Strahlregelung, wobei der Fokus entsprechend der Kapselgeometrie synchron zur Drehung nachgeführt wird, oder, ganz besonders bevorzugt mit einer Intensitätsregelung, die eine Defokussierung durch Leistungserhöhung ausgleicht.

Vor dem eigentlichen Schweißvorgang müssen vorbereitende Maßnahmen getroffen werden. Diese ergeben sich aus der Berücksichtigung von Schweißanforderungen hinsichtlich der verwendeten Energiequelle, der Konstruktion der Kapseln in geometrischer Hinsicht und hinsichtlich der Materialwahl sowie der Auswahl von Zuschlagstoffen, z.B. Farbstoffen, die einem oder mehreren Kapselteilen zugesetzt werden können. Letztere werden bevorzugt beim Laserverschweißen verwendet.

Der Vorteil der Farbstoffe im Kunststoff besteht darin, daß die Farben das Laserlicht im Kunststoff absorbieren und dadurch den Kunststoff lokal soweit erwärmen, daß es zu einer Verschweißung kommt.

Dabei werden die verwendeten Farbstoffe auf die Frequenz des Laserlichts und die benötigte Wärmemenge eingestellt.

Als Farbstoffe werden Farbstoffe gewählt, die die pharmazeutische Qualität der Formulierung in der Kapsel nicht verändern. Bevorzugt werden Lebensmittelfarben verwendet.

Beispiele für besonders bevorzugte Farbstoffe sind:

| Farbstoff | Farbe |
|---|---|
| Iron Oxide Red = E 172 =Fe₂O₃ | rot |
| FD&C Red 3 = E 127 = C₂₀H₆l₄O₅Na₂*H₂O = Erythrosi | rot |
| beta-Carotin = E 160a = C₄₀H₅₆ | gelb |
| Iron Oxide Yellow = E 172 = FeO(OH) | gelb |
| FD&C Blue 2 = E 132 = C₁₆H₈N₂O₈S₂Na₂ = Indigotin | blau |
| Chlorophyllin= E 141 = C₃₄H₃₁N₄O₆CuNa₃ oder C₃₄H₃₁N₄O₆CUK₃ | grün |
| Caramel = E 150a oder E 150d = "abgebräunter" Zucker | beige |
| Titanidioxid = E 171 | weiß |

Darunter sind die anorganischen Pigmente besonders bevorzugt.

Die Zuschlagstoffe können entweder auf die fertigen Kapselteile aufgetragen (z.B. durch Sprühen, Drucken, Pinseln, Tauchen) und dann beim Schweißprozeß aufgeschmolzen werden, oder sie werden bereits bei der Herstellung der Kapseln z.B. in einem Masterbatchverfahren mit in das Kapselmaterial eingearbeitet.

Beim Laserverschweißen muß für eine vollständige Verschweißung des gesamten Kapselumfangs der Umfang vollständig bestrahlt werden. Möglich ist eine Linienoptik, die gleichzeitig den kompletten Umfang verschweißt. Bevorzugt ist eine relative Bewegung von Kapsel und Lichtstrahl und die damit verbundene Führung des Brennflecks entlang dem Kapselumfang. Beispielsweise kann dies durch Rotation der Kapsel im Lichtstrahl des Lasers erfolgen.

Alternativ kann durch rotierende Spiegel der Laserstrahl um die Kapsel herumgeführt werden. Besonders bevorzugt ist dabei die relative Drehung von Kapsel und Laserstrahl mittels einer Halterung, die eine Synchronisation zwischen Drehwinkel und Laseraktivität ermöglicht.

Als Meßeinrichtung zur rückgekoppelten Steuerung des Schweißorgans wird beim Laserverschweißen bevorzugt eine Strahlungsmeßeinrichtung verwendet.
Die Rückkopplung gleicht Schwankungen z.B. der Materialstärke, des Reflexionsvermögens, der Fokussierung und anderer Umwelteinflüsse aus, indem entweder die Drehgeschwindigkeit oder bevorzugt die Laserleistung nachgeregelt wird. In diesem Zusammenhang wird bevorzugt ein möglichst kleiner Fleck des Nahtmaterials unter Temperatur- bzw. Strahlungsmessung aufgeschmolzen. Durch die oben beschriebene Relativdrehung der Kapsel um den Laserstrahl wird dann die Schweißnaht erzeugt.

Die für das Verschweißen notwendige Laserleistung hängt von der gewählten Optik ab (Größe des Brennflecks), von der Vorschubgeschwindigkeit, von der Oberflächenbeschaffenheit des Materials (z.B. Rauhigkeit), von den optischen Eigenschaften des Materials (z.B. Transparenz) und von der notwendigen Schmelztemperatur des Materials. Beispielsweise wurden grün gefärbte Kunststoffkapseln mit einem 5 Watt Argon-lonenlaser-Strahl geschweißt (Wellenlänge all line, 514 nm und 488 nm), die Fokussierung erfolgte mit einem Mikroskopobjektiv (Vergrößerung 10X). Dieselben Kapseln konnten auch mit einem Infrarotlaser (Wellenlänge um 900 nm) bei einer Strahlleistung von ca. 100 Watt verschweißt werden

Durch das Laserschweißverfahren kann sowohl sofort als auch erst beim zweiten oder noch späteren, wiederholten Bestrahlen die Schweißnaht ausgebildet werden. Bei Wiederholung werden die Kapsel und Lichtquelle bevorzugt so relativ zueinander rotiert, daß die einmal bestrahlte Stelle noch nicht wieder erkaltet ist, wenn sie erneut auf den Laserstrahl trifft. Der Fokus des Laserstrahls wird bevorzugt gerade so groß gewählt, daß nur wenig Wärme an der Innenseite der Kapselwand und lokalisiert auf die Naht der Kapsel erzeugt wird.

Die Breite der Schweißnaht kann durch schnelle schwingende Bewegung des Brennfleckes in andere Richtungen als der Schweiß-Vorschubrichtung eingestellt werden. Bevorzugt ist die Breiteneinstellung durch optische Abbildung der Laserstrahlung auf einen Brennfleck, bevorzugt von kleiner 1 mm. Besonders bevorzugt ist ein Brennfleckdurchmesser von weniger als 0.5 mm.

Beim Heißgas-Schweißen erfordert die vollständige Verschweißung des gesamten Kapselumfangs die gleichmäßige Erhitzung der Kapsel entlang ihres Umfangs. Dies kann durch eine oder mehrere beispielsweise sichel- oder ringförmige Düse(n) ermöglicht werden, die gleichzeitig den kompletten Umfang verschweißt (verschweißen). Bevorzugt ist jedoch eine relative Bewegung von Kapsel und einem Flachstrahl der durch eine entsprechende Düse mit flacher rechteckiger Öffnung erzeugt wird. Die dadurch geschaffene Schmelzzone kann dann entlang dem Kapselumfang bis zur vollständigen Verschweißung geführt werden. Besonders bevorzugt ist die Drehung der Kapsel mittels einer Halterung, die eine Synchronisation zwischen Drehwinkel und Heißgaszutritt ermöglicht.

Bei Einzelstückfertigung sind eine Düse oder mehrere Düsen mit kleinem Querschnitt vorteilhaft.

Bei industrieller Serienfertigung ist eine (oder sind zwei) lange Schlitzdüse(n) einzusetzen, an der (an denen) auch mehrere Kapseln auf einmal unter kontinuierlicher Drehung vorbeigeführt werden.

Im Fall des Heißgasverschweißens können sichelförmige Schlitzdüsen vorteilhaft verwendet werden, wenn das Verfahren dergestalt ist, daß die Kapseln im Wärmestrahl gedreht werden.

Die Maße der Schlitze der Düse werden so gewählt, daß ein bezüglich der Höhe sehr enger Wärmestrahl entsteht. Bevorzugt beträgt die Höhe der Schlitzöffnung bis zu 3 mm, bevorzugt bis zu 2 mm, besonders bevorzugt bis zu 1 mm. Die Länge der Öffnung ist variabel. Im Fall von nicht radialen Schweißnähten sollte die Länge ebenfalls wie die Höhe auf 3 mm, bevorzugt 2 mm, besonders bevorzugt 1 mm begrenzt sein. Im Fall von radial oder spiralförmig um die Kapsel verlaufenden Schweißnähten ist die Länge des Düsenschlitzes von untergeordneter Bedeutung

Die Höhe der Schweißnaht wird von der Höhe des Düsenstrahls bestimmt. Im Fall einer radial um die Kapsel verlaufenden Schweißnaht wird der Bereich der Kapsel an dem die Schweißnaht ausgebildet werden soll parallel der Längsseite der Düsenordnung im Energiestrahl geführt. Die Länge der Düsenordnung und die Temperatur des Energiestrahls bestimmen zusammen mit dem Schmelzbereich des Kapselmaterials die Verweildauer der Kapsel im Energiestrahl. D.h. je länger die Düsenöffnung und je heißer der Energiestrahl, um so schneller muß die die Kapsel parallel zu dieser Richtung gedreht werden. D.h. die Schweißnaht um die Kapsel wird parallel zur Länge der Düsenordnung ausgebildet.

In solchen Fällen kann die Länge der Schlitzdüse viele cm betragen oder sogar in den Meterbereich liegen. Ein solche langgezogene Schlitzdüse ist besonders dann vorteilhaft, wenn mehrere Kapseln hintereinander fließbandartig mit einer Schweißnaht versehen werden sollen.

Das Verfahren wird bevorzugt so gesteuert, daß die Schweißnaht nicht sofort entsteht, sondern erst beim zweiten oder noch späteren, wiederholten Anblasen mit Heißgas. Die Wiederholung erfolgt bevorzugt durch Rotation von Kapsel und Heißgasdüse relativ zueinander, ehe die angeblasene Stelle wieder erkaltet ist.

Die für das zum Verschweißen notwendige Temperatur des Gases hängt von den Schmelzbereichen der verwendeten Kunststoffe und von deren Entfernung zur Heißgasdüse ab. Um jedoch einen möglichst schmalen Heißgasstrahl auf die Nahtstelle aufzutragen, wird die Kapsel sehr dicht an der Düse vorbeigeführt. Bevorzugt in einer Entfernung von 5 mm.

Die Breite des Heißgasstrahls auf der Schweißstelle beträgt 1 mm bis 2 mm.

Ein weiterer Aspekt der vorliegenden Erfindung betrifft neben dem Schweißverfahren und der dadurch erhältlichen Kapsel eine an das Schweißverfahren angepaßte Kapselhalterung.

Die Kapselhalterung besteht aus zwei voneinander getrennten Formen, deren Innengestaltung schalenförmig ist. Die Schalenform ist jeweils so ausgebildet, daß die eine den Kapselkörper schlüssig aufnimmt, die andere die Kapselkappe, wobei im geschlossenen Zustand lediglich der zu verschweißende Nahtbereich von der Halterung unbedeckt bleibt.

Der Vorteil dieser Maßnahme ist, daß nur der zu verschweißende Bereich der Kapsel mit dem Energiestrahl in Berührung kommen kann und die anderen Bereiche der Kapsel vor dem Strahl geschützt sind.

Bevorzugt werden von der als Naht auszubildenden Kante der jeweiligen Kapselhälften bis zu 3 mm frei gelassen, ganz besonders bevorzugt 0.5 mm bis1 mm.

Die äußere Gestaltung der Halterung ist unkritisch, soll jedoch den Abfluß des Heißgases ermöglichen. Beispielsweise können die beiden Halterungsteile von zylindrischer Form sein.

Das Oberteil und das Unterteil der Kapselhalterung sind dabei so gekoppelt, daß Position der Werkstücke auch bei angeschmolzener Verbindung zwischen den Einzelteilen gehalten wird, ohne die Kapsel einer Torsion oder Spannung auszusetzen. Dies ist besonders wichtig für den Schweißvorgang, wenn die Formstabilität der Kapsel durch Erwärmen eines Teilbereichs verringert wird.

Die Aufgabe der Kapselhalterungen besteht darin, die Kapsel zu halten und sie an die Energiequelle für den Verschweißungsvorgang heran zu führen. Gleichzeitig dienen sie der Kühlung der Bereiche der Kapselwände, die nicht verschweißt werden sollen und damit auch dem Schutz der Formulierung in der Kapsel.

Um die Formulierung gegenüber den Bedingungen des Schweißvorgangs zu schützen, liegt die Füllhöhe der Kapsel bevorzugt unterhalb der Schweißnaht, in dem geschützten Bereich.

Die Kapselhalterungen können so ausgebildet sein, daß sie die Kapsel kühlen. Hierfür eignen sich beispielsweise Systeme mit Wasserkühlung oder Peltier-Elemente.

Die Kapselhalterung kann aus Metall, z.B. Aluminium, Kupfer, Edelstahl oder aus hitzebeständigem Kunststoff sein, z.B. Polytetrafluorethylen (=Teflon®).

Bevorzugt ist wenigstens ein Teil der beiden Hälften der Kapselhalterung mit einer Drehvorrichtung verbunden, so daß die Kapsel um die Energiequelle gedreht werden kann. Bevorzugt dreht sich die Kapselhalterung selbst um die eigene Achse, die durch das Oberteil und Unterteil der Halterung aufgespannt wird.

Mehrere Kapselhalterungen können auf einer Fördervorrichtung beispielsweise und bevorzugt in Reihe angeordnet sein. In diesem Fall ist jeweils die Halterung für den Kapselkörper auf einer Fördervorrichtung verbunden. Darüber läuft parallel eine zweite Fördervorrichtung, an der die Halterung für die Kapselkappe so befestigt ist, daß jeweils eine Kapsel mit ihrem Körper in der dafür vorgesehenen Halterung ist und die Kapselkappe in der für sie vorgesehenen Halterung. Die Fördervorrichtungen sind dergestalt, daß die beiden Hälften der Kapselhalterungen nach dem Verschweißen auseinander geführt werden können, so daß die Kapseln nur in einer der beiden Hälften verbleiben, bevorzugt derjenigen für den ursprünglichen Kapselkörper. Dies kann z.B. dadurch geschehen, daß die Fördereinrichtungen jeweils Fließbänder sind, die sich nach dem Ort des Verschweißens zunächst innerhalb der gleichen Ebene divergierend auseinanderlaufen. Danach können die Kapseln aus der Halterung entnommen werden.

In einer anderen Ausführungsform können die Halterungen über einen teleskopartig ausfahrbaren Arm an der Fördervorrichtung befestigt sein. Diese Arme sind in Richtung des jeweils anderen Arms ausgefahren, wenn die Kapsel verschweißt wird, so daß die Kapsel von den beiden Antipoden der Halterung in der oben beschriebenen Weise geschützt vorliegt. Nach dem Schweißvorgang ziehen sich die Arme wieder zusammen, so daß die beiden Hälften der Halterung auseinander bewegt werden.

Auf diese Weise können die Kapseln an die Energiequelle herangeführt werden.

Bevorzugt sind die Halterungen um sich selbst drehbar auf der Fördervorrichtung gelagert. Sobald die Kapsel in den Energiestrahl hinein gefördert worden ist, dreht sich die Kapselhalterung als Ganzes um sich selbst und dreht dadurch die Kapsel im Energiestrahl, so daß die Schweißnaht rund um die Kapsel ausgebildet wird.

Bei Kapselhalterungen die nicht drehbar gelagert sind, kann der Energiestrahl entsprechend um die Kapsel geführt werden.

Während des Verschweißens bildet sich durch das Erwärmen in der Kapsel ein Überdruck aus. Dieser Druck stellt ein erhebliches Risiko dar, da er bei zu starker Erwärmung zu Blasenbildung in der erwärmten Wandung der Kapsel führen kann.
Die erfindungsgemäße Kapselhalterung führt auch bezüglich dieser Gefahrenquelle vorteilhaft zur Abhilfe.

Zusätzlich schützt die Kapselhalterung auch die Kapselteile, die nicht verschweißt werden sollen sowie die Formulierung in der Kapsel.

Durch das Verfahren entstehen Arzneimittel-haltige Kapseln für Inhalatoren, die vollständig dicht sind, so daß das Arzneimittel nicht ohne Zerstörung der Kapsel aus dieser austreten kann.

Der Vorteil der erfindungsgemäßen Kapseln besteht darin, daß sie eine sehr geringe Wasserdampfpermeabilität aufweisen, insbesondere an der Nahtstelle und dadurch auch in den verschiedenen Klimazonen, z.B. in der Klimazone 3 mit hoher Luftfeuchtigkeit verwendet werden können, ohne daß die pharmazeutische Qualität der Formulierung darunter leidet. Diese Kapseln besitzen auch in anderen verschiedenen Stadien des Lebens der Kapsel von der Herstellung bis zur Verwendung diverse Vorteile in Bezug auf die Verwendungsfähigkeit der Kapsel als Träger von pharmazeutischen Zubereitungen, die Art der Verabreichung der Inhaltsstoffe, die Haltbarkeit der Inhaltsstoffe und/oder die Verwendungsfähigkeit der Kapsel in bestimmten Ländern. Ein weiterer Vorteil der erfindungsgemäßen Kapselmaterialien besteht z.B. darin, daß sie dazu neigen, pulverförmige Stoffe nicht an sich zu binden, so daß ein exaktes Dosieren der lungengängigen Feinfraktion erleichtert wird.

Diese Kapseln können auch in nicht-medizinischen Aerosolen eingesetzt werden.

### Beschreibung der Abbildungen

Die Abbildungen zeigen exemplarisch verschiedene Ausführungsformen der erfindungsgemäßen Kapsel, dienen jedoch nur zur Illustration ohne den Umfang der Erfindung einzuschränken.
Fig. 1 zeigt die einfachste und bevorzugteste Ausführungsform der erfindungsgemäßen Kapsel im lateralen Querschnitt.
Fig. 2a und 2b zeigen je eine unterschiedliche Ausführungsform der Kapsel mit sich verjüngendem Wulst am Körper im lateralen Querschnitt.
Fig. 3 zeigt eine Ausführungsform der Kapsel mit kantenförmigem Wulst am Körper im lateralen Querschnitt.
Fig. 4 zeigt eine Ausführungsform der Kapsel mit sich verjüngendem Wulst am Körper und ringförmiger Vertiefung an Körper und Kappe im lateralen Querschnitt.
Fig. 5 zeigt eine Ausführungsform der Kapsel mit sich verjüngendem Wulst am Körper und ringförmiger Vertiefung an Körper und Kappe in Frontalansicht.
Fig. 6 zeigt eine Ausführungsform der Kapsel mit sich verjüngendem Wulst am Körper und punktförmigen Vertiefungen bzw. Erhebungen an Körper und Kappe in Frontalansicht.
Fig. 7 zeigt eine Ausführungsform der Kapsel mit sich verjüngendem Wulst am Körper und punktförmigen Erhebungen am Körper und punktförmigen Löchern in der Kappe in Frontalansicht.
Fig. 8 zeigt eine Ausführungsform der Kapsel mit Rippen am Körper in Frontalansicht.
Fig. 9 zeigt die Kapsel der Fig. 7 im horizontalen Querschnitt.
Fig 10a, 10b und 10c zeigen Ausführungsformen der Kapsel mit je verschiedenem Querschnitt.
Fig. 11 a - g zeigen verschiedene Ausführungsformen von Kapseln 1 mit nicht mittig-angeordneter Verschlußstelle zwischen die Kappe mit dem Körper zur Ausbildung der Schweißnaht 13.
Fig. 12 a - h zeigen Kapseln mit verschiedenen Formen der Schweißnaht (geradlinig, spiralförmig, meanderförmig, zick-zackförmig, Punktverschweißungen, parallele diagonal verlaufende nicht durchgehende Schweißnähte).
Fig. 13 zeigt eine Kapselhalterung.
Fig. 14 zeigt eine Kapselhalterung im Energiestrahl eines Lasers.
Fig. 15 zeigt eine Kapselhalterung mit einer Kapsel, um die herum der Laserstrahl geführt wird.
Figur 16 zeigt eine Kapselhalterung im Energiestrahl einer Heißgasdüse.
Fig. 17 zeigt eine Kapsel, bei der die Nahtstelle zwischen Kappe 2 und Körper 3 senkrecht zur Längsachse der Kapsel ausgebildet ist.
Fig. 18 zeigt eine Kapsel bestehend aus zwei Kappen 2 und einem Körper 3.

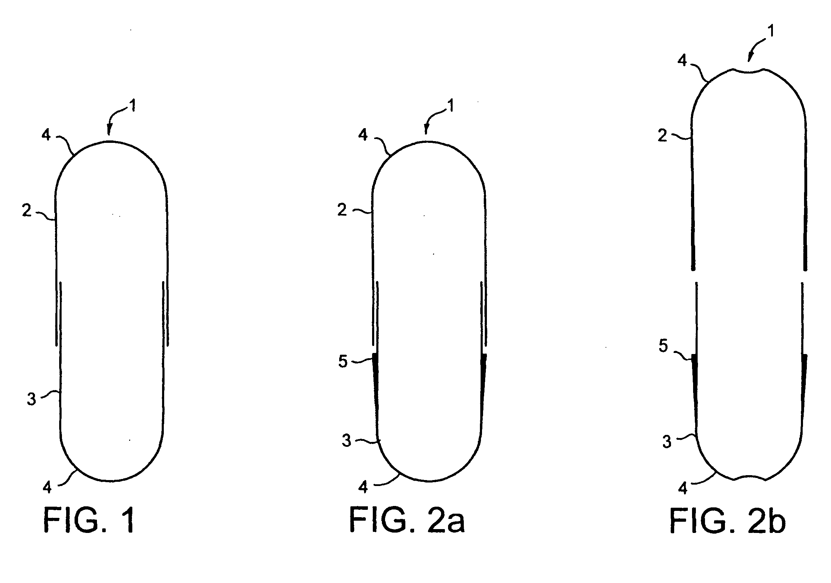

Ein Ausführungsbeispiel mit einer kugelförmigen Kapsel ist nicht dargestellt. In Figur 1 ist die einfachste Ausführungsform der erfindungsgemäßen Kapsel 1 im Querschnitt gezeigt. Die Kapsel 1 besteht aus der Kappe 2 und dem Körper 3, die teleskopartig ineinander gesteckt sind. Kappe 2 und Körper 3 sind von gleicher Gestalt und haben je eine konvexe Unterseite 4.
In Figur 2a ist im Querschnitt eine Ausführungsform gezeigt, bei der am Körper 3 der Kapsel 1 ein Wulst 5 ausgebildet ist, der sich zum geschlossenen Ende des Körpers hin verjüngt. Mit der zum offenen Ende des Körpers hin orientierten Seite steht der Wulst 5 nahezu senkrecht auf dem Körper. Die so ausgebildete Kante begrenzt den Bereich des Körpers über den die Kappe 2 teleskopartig geschoben werden kann.

Eine andere Ausführungsform ist in Figur 2b abgebildet. Der Querschnitt zeigt, daß sich diese Ausführungsform von der in Figur 2a dargestellten dadurch unterscheidet, daß die Wandstärke der Kappe 2 bzw. des Körpers 3 nicht über den gesamten Bereich gleich stark ausgebildet ist, sondern über einzelne Teilbereiche variiert. Zusätzlich weisen die konvexen Unterseiten 4 der Kappe bzw. des Körpers je eine konkave Einbuchtung am Scheitelpunkt auf.

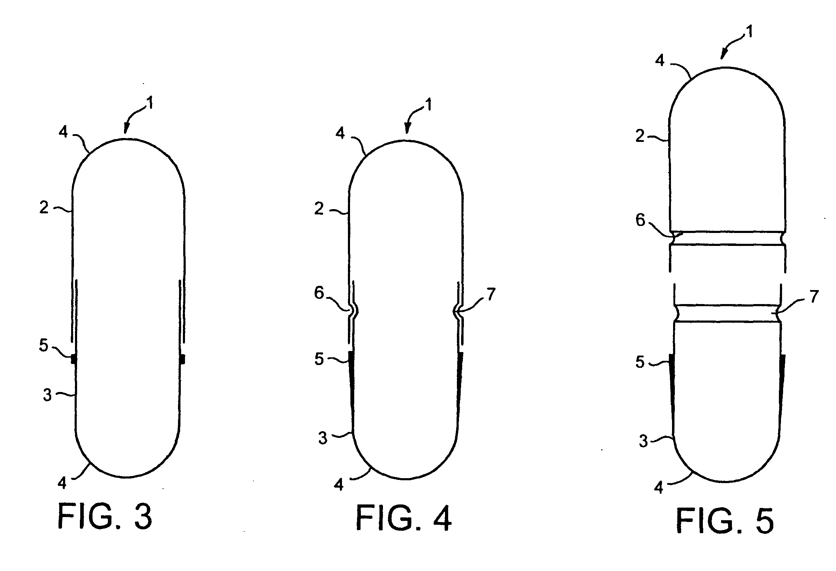

In Figur 3 ist eine Ausführungsform dargestellt, bei der der Wulst 5 sowohl zur Oberseite des Körpers als auch zu seiner Unterseite nahezu rechtwinkelig auf dem Körper aufsitzt.
Die Ausführungsform der Figur 4 stellt eine Weiterentwicklung der Ausführungsform der Figur 2a dar, bei der eine ringförmige Vertiefung 6 bzw. 7 in Kappe 2 bzw. Körper 3 zum besseren Verschluß der Kapsel 1 ausgebildet ist.
In Figur 5 ist eine Frontalansicht der in Figur 4 als Querschnitt gezeigten Ausführungsform abgebildet.

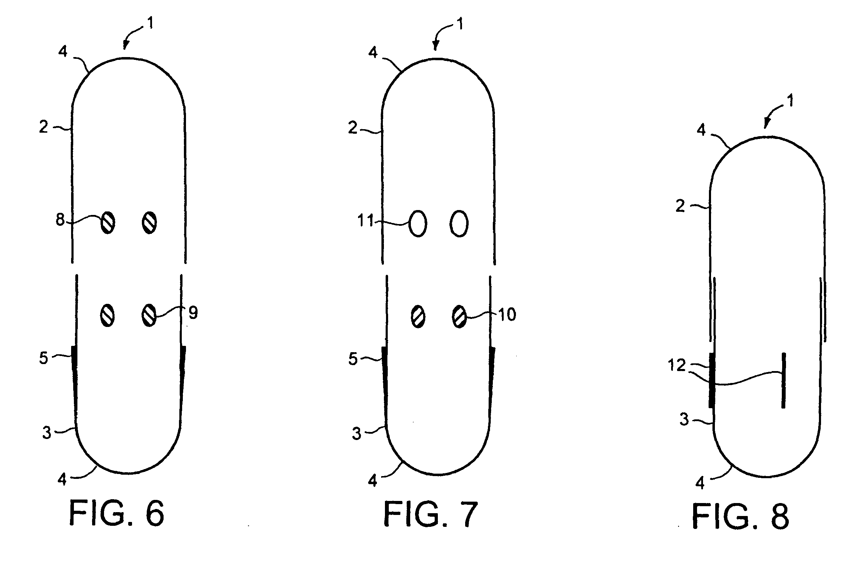

Figur 6 zeigt eine weitere Variante der Erfindung mit punktuellen Vertiefungen 8 und 9 als Frontalansicht.
In Figur 7 ist eine Variante der Kapsel 1 dargestellt, bei der am Körper 3 nahe dem offenen Ende Erhebungen 10 und in der Kappe 2 nahe dem offenen Ende Löcher 11 so ausgebildet sind, daß die Erhebungen 10 beim Verschließen der Kapsel in die Löcher 11 einrasten.
Die Figur 8 stellt eine Ausführungsform der Kapsel 1 von außen dar, bei der auf dem
Körper 3 die Rippen 12 ausgebildet sind.

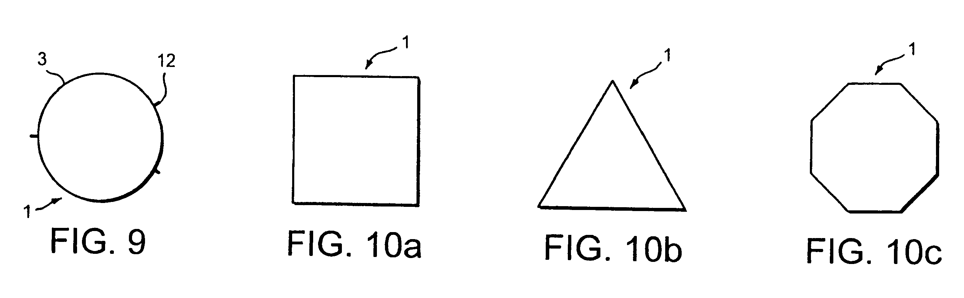

Figur 9 zeigt den Körper 3 der Ausführungsform der Figur 7 im Querschnitt. Der Querschnitt zeigt, daß die drei Rippen 12 nicht rotationssymmetrisch um die Mittelachse des Körpers angeordnet sind. In Figur 10a ist eine Kapsel 1 mit viereckigem Querschnitt dargestellt, in Figur 10b eine mit dreieckigem und in Figur 10c eine mit achteckigem Querschnitt.

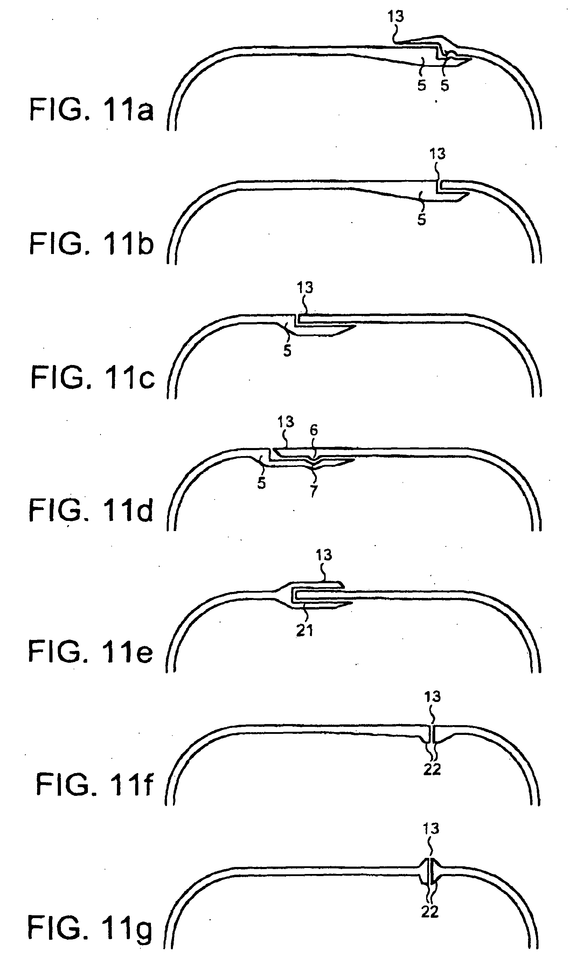

Fig. 11 a - g zeigen verschiedene Ausführungsformen von Kapseln 1 mit nicht mittig-angeordneter Verschlußstelle zwischen der Kappe mit dem Körper zur Ausbildung der Schweißnaht 13. Fig. 11a zeigt einen Verschluß bei dem auf der Außenseite des Körpers eine nach innen versetzte Kante (5) ausgebildet ist und auf der innenseite der Kappe eine nach außen gerichtete Kante (5). Die Verbindung wird durch eine Raste, bevorzugt zwei Rasten verstärkt. Fig. 11b zeigt eine Ausführungsform, bei der nur der Körper eine nach innen versetzte Kante (5) aufweist. Kappe und Körper schließen stufenlos gegeneinander ab. Fig. 11c zeigt eine Ausführungsform, bei der nur an der Kappe eine nach innen versetzte Kante (5) ausgebildet ist. Der Körper weist dieses Merkmal nicht auf. Kappe und Körper schließen stufenlos gegeneinander ab. Fig. 11d zeigt eine analoge Ausführungsform, bei der im überlappenden Bereich von Kappe und Körper eine oder mehrere Noppen und dazu eine oder mehrere passende Vertiefung(en) auf dem Gegenstück ausgebildet ist (sind), um einen besseren vorläufigen Verschluß vor dem Verschweißen zu erreichen. Fig. 11e weist eine Ausführungsform auf, bei der der Verschluß dergestalt ist, daß der Körper keine Verschlußmerkmale aufweist, die Kappe an ihrem offenen Ende einen U-förmigen Umlauf (21) aufweist, der auf die offene Kante der Körperwandung aufgesteckt werden kann. Fig. 11f und g weisen Ausführungsformen auf, bei denen sich die Dicke der Wandungen die offenen Enden (22) von Körper und Kappe verdicken um eine breitere Berührungszone im Verschlußbereich zwischen den beiden Kapselhälften zu erreichen.

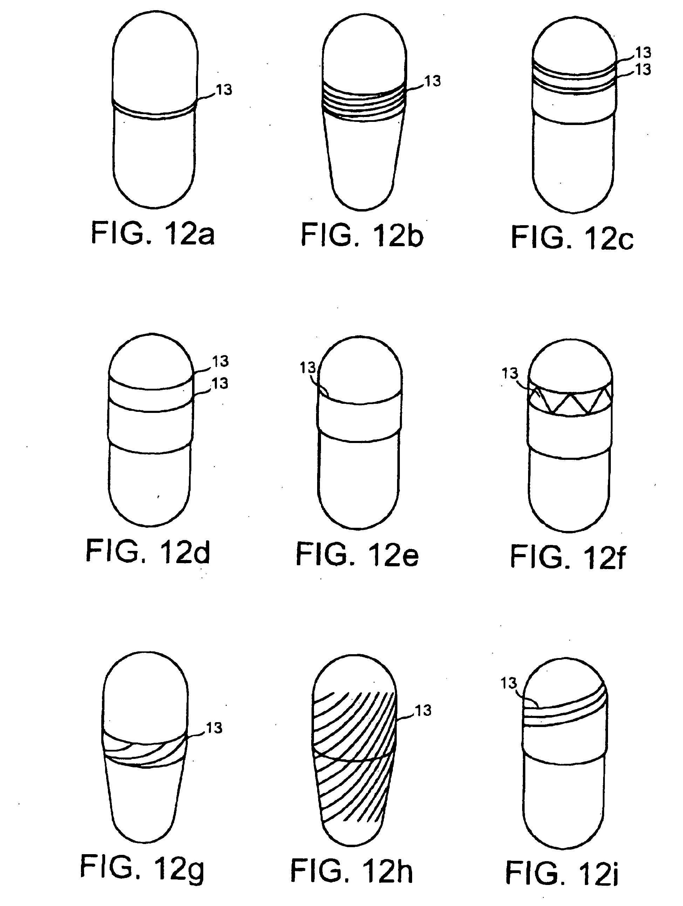

Fig. 12 a - i zeigen Kapseln 1 mit verschiedenen Formen der Schweißnaht 13: a) c) und d) geradlinig, e) Punktverschweißungen, f) zick-zackförmig, g) und h) parallele diagonal verlaufende nicht durchgehende Schweißnähte, b), i) spiralförmig.

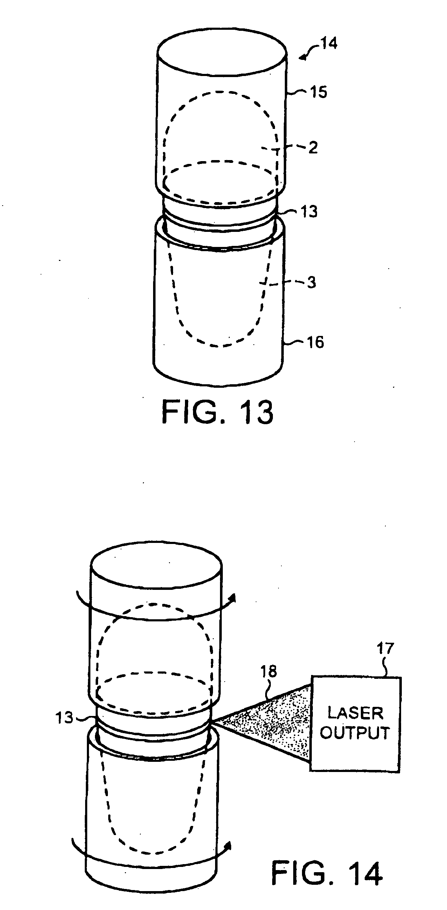

Fig. 13 zeigt eine Kapselhalterung 14 bestehend aus der Halterung 15 für den Kapselkörper 3 und der Halterung 16 für die Kapselkappe 2.
Fig. 14 zeigt eine Kapselhalterung 14 im Energiestrahl 18 eines Lasers 17.

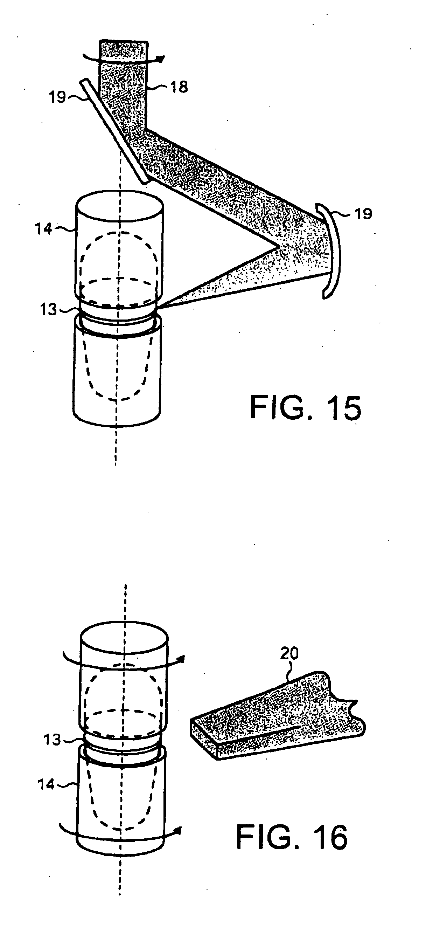

Fig. 15. zeigt eine Kapselhalterung 14 mit einer Kapsel 1, um die herum der Laserstrahl 18 mittel zweier Spiegel 19 geführt wird.
Figur 16. zeigt eine Kapselhalterung 14 im Energiestrahl einer Heißgasdüse 20. Die Düse kann auch breiter ausgebildet sein, damit an der schlitzförmigen Düse gleichzeitig mehrere Kapseln in Reihe vorbeigeführt werden können.

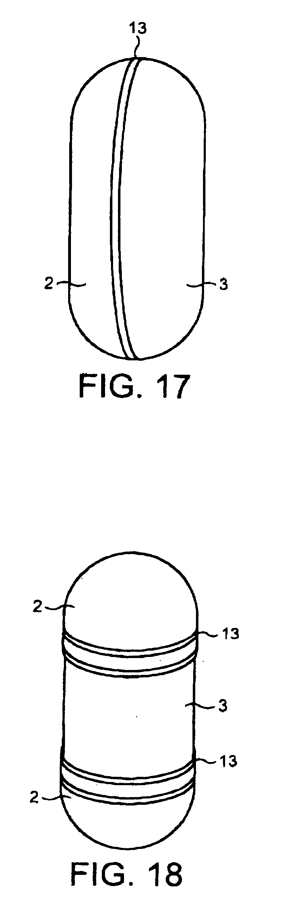

Fig. 17 zeigt eine Kapsel, bei der die Nahtstelle zwischen Kappe 2 und Körper 3 senkrecht zur Längsachse der Kapsel ausgebildet ist.
Fig. 18 zeigt eine Kapsel bestehend aus zwei Kappen 2 und einem Körper 3. In diesem Fall stellt der Körper ein nach zwei Seiten offenes Rohr dar, wobei jede Öffnung von jeweils einer Kappe 2 verschlossen ist.
Bei allen Ausführungsformen können die zum Verschließen der Kapsel beschriebenen Merkmale in Bezug auf Kappe und Körper auch reziprok angeordnet sein, d.h. Verschlußmerkmale die sich an der Kappe befinden können an dem Körper ausgebildet sein und umgekehrt.

### Beispiele

### Typische Betriebsdaten für Heißluftgebläse:

Steinel Heißluftgebläse 1800 W, elektronisch geregelt. Spezialdüse mit Düsenöffnung 1 mm x 7 mm, Heißlufttemperatur ca. 250 C am Düsenaustritt. Drehung der Kapsel durch Schrittmotor langsamer als 3 Umdrehungen/Sekunde in einem Abstand von 10 mm.

### Betriebsdaten für Laser

Die Betriebsdaten werden durch den jeweiligen Laser vorgegeben. Erreicht werden 0.75 Sekunden pro Kapsel bei ca. 30 Watt Lichtleistung.

Die mittlere Verweildauer einer Schweißstelle beträgt im Fall des Laserschweißens bei einem Brennfleck von 0.1 mm Durchmesser, wenn die Kapsel 2 Umdrehungen innerhalb von 8 Sekunden durchführt, was einer Umfangsgeschwindigkeit von 4,6 mm pro Sekunde entspricht näherungsweise 22 Millisekunden. Dabei wurde eine Kapsel mit einer Länge von 15,9 mm, einem Durchmesser des Kapselkörpers von 5,57 mm und einen Durchmesser der Kapselkappe von 5,83 mm verwendet, die Wandstärke des Kapselkörpers betrug 0,25 mm und die der Kapselkappe 0,35 mm.

Im Fall eines 1.5 Watt Argon lonenlasers wird eine grüne Kapsel verwendet.

Im Fall des Heißgasverschweißens ergibt sich für die gleiche Kapsel eine mittlere Verweildauer von ca 0,26 Sekunden, wenn ein ca. 1 mm hoher und 7 mm breiter Heißluftstrahl verwendet wird und die Kapsel innerhalb von 8 Sekunden 12 mal in dem Strahl gedreht wird. Die Geschwindigkeit am Kapselumfang beträgt dann ca. 27 mm pro Sekunde.

### Beispiele für Kapseln:

Länge der Kapselkörper: 22,2 ±0,46 mm; 20,22 ±0,46 mm; 20,98 ±0,46 mm; 18,4 ±0,46 mm; 16,61 ±0,46 mm; 15,27 ±0,46 mm; 13,59 ±0,46 mm; 12,19 ±0,46 mm; 9,3 ±0,46 mm.

Länge der Kapselkappe: 12,95 ±0,46 mm; 11,74 ±0,46 mm; 11,99 ±0,46 mm; 10,72 ±0,46 mm; 9,78 ±0,46 mm; 8,94 ±0,46 mm; 8,08 ±0,46 mm; 7,21 ±0,46 mm; 6,2 ±0,46 mm.

Äußerer Durchmesser der Kapselkörper: 9,55 mm; 8,18 mm; 7,36 mm; 7,34 mm; 6,63 mm; 6,07 mm; 5,57 mm; 5,05 mm; 4,68 mm.

Äußerer Durchmesser der Kapselkappen: 9,91 mm; 8,53 mm; 7,66 mm; 7,64 mm; 6,91 mm; 6,35 mm; 5,83 mm; 5,32 mm; 4,91 mm.

Gesamtlänge der geschlossenen Kapsel: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3 ±0,3 mm; 11,1 ±0,3 mm.

Kapselvolumina: 1,37 ml; 0,95 ml; 0,78 ml; 0,50 ml; 0,37 ml; 0,30 ml; 0,21 ml; 0,13 ml.

Gewicht der Kapseln: 163 mg; 118 mg; 110 mg; 96 mg; 76 mg; 61 mg; 48 mg; 38 mg; 28 mg.

## Patentansprüche

1. Kapsel für Inhalatoren enthaltend Arzneimittel für die Inhalation, wobei die Kapsel aus wenigstens einer Kapselkappe und einem Kapselkörper und optional weiteren Teilelementen besteht, die teleskopartig ineinander eingesetzt werden, die Teilelemente stoffschlüssig verschweißt sind und im überlappenden Bereich oder in den überlappenden Bereichen der Teilelementen je eine Schweißnaht ausgebildet ist, und wobei die Kapsel nach einem Verfahren herstellbar ist, bei dem
- die Kapselkappe und der Kapselkörper von einer Kapselhalterung aus zwei zu einander synchron führbaren Teilen gehalten werden,
- ein Teil der Kapselhalterung die Kapselkappe und der andere Teil der Kapselhalterung den Kapselkörper formschlüssig so umgeben, dass der überlappende Bereich nicht bedeckt wird, an dem die Schweißnaht ausgebildet werden soll, die anderen Bereiche der Kapselkappe und des Kapselkörpers jedoch von der Kapselhalterung bedeckt werden und
- die so geschützte geschlossene Kapsel in einen Energiestrahl aus Heißgas oder Laserlicht gebracht wird, und
- der Energiestrahl in einer Relativbewegung zur Kapsel wenigstens einmal vollständig um die Senkrechtachse des überlappenden, von der Kapselhalterung nicht geschützten Bereichs der Kapselelemente herumgeführt wird oder
- in dem Energiestrahl die Kapsel durch die beiden synchron bewegten Teile der Kapselhalterung ohne Torsionsspannung oder Scherspannung geführt wird, so dass das Kapselmaterial in diesem Bereich angeschmolzen, aber nicht zerstört wird und der überlappende Bereich durch eine stoffschlüssige Nahtstelle dicht versiegelt wird.

## Claims

1. Capsule for inhalers containing medicaments for inhalation, wherein the capsule consists of at least one capsule cap and a capsule body and optionally other partial elements which are inserted telescopically into one another, the partial elements are materially welded together and a weld seam is formed in the overlapping area or in the overlapping areas of the partial elements, and wherein the capsule can be produced by a process wherein
- the capsule cap and capsule body are held by a capsule holder consisting of two parts which can be guided synchronously with one another,
- one part of the capsule holder interlockingly surrounds the capsule cap and the other part of the capsule holder interlockingly surrounds the capsule body so that the overlapping area on which the weld seam is to be produced is not covered, but the other regions of the capsule cap and the capsule body are covered by the capsule holder, and
- the sealed capsule thus protected is brought into an energy flow of hot gas or laser light, and
- the energy flow is guided at least once right round the vertical axis of the area of overlap of the capsule elements not protected by the capsule holder in a relative movement to the capsule, or
- in the energy flow the capsule is guided by the two synchronously moving parts of the capsule holder without any torsional stress or shear stress so that the capsule material in this region begins to melt but is not destroyed and the area of overlap is tightly sealed by a seam formed in the material.

## Revendications

1. Capsule pour inhalateurs contenant des médicaments pour l'inhalation, où la capsule consiste en au moins une coiffe de capsule et un corps de capsule et éventuellement d'autres éléments partiels qui sont insérés les uns dans les autres de manière télescopique, les éléments partiels sons soudés par assemblage de matière et un cordon de soudure est formé dans le domaine chevauchant ou dans les domaines chevauchants des éléments partiels, et où la capsule peut être produite selon un procédé dans lequel
- la coiffe de capsule et le corps de capsule sont maintenus par un dispositif de maintien de capsule constitué par deux parties qui peuvent être conduites l'une vers l'autre de manière synchrone,
- une partie du dispositif de maintien de capsule entoure par assemblage de forme la coiffe de capsule et l'autre partie du dispositif de maintien de capsule entoure par assemblage de forme le corps de capsule de telle manière que le domaine chevauchant au niveau duquel le cordon de soudure doit être formé n'est pas couvert tandis que les autres domaines de la coiffe de capsule et du corps de capsule sont couverts par le dispositif de maintien de capsule et
- la capsule fermée ainsi protégée est amenée dans un faisceau d'énergie constitué par un gaz chaud ou de la lumière laser, et
- le faisceau d'énergie est entraîné dans un mouvement relatif par rapport à la capsule au moins une fois totalement autour de l'axe vertical du domaine chevauchant des éléments de capsule qui n'est pas protégé par le dispositif de maintien de capsule ou
- dans le faisceau d'énergie la capsule est conduite par les deux parties du dispositif de maintien de capsule mues de manière synchrone sans tension de torsion ni tension de cisaillement de sorte que la matière de la capsule est fondue dans ce domaine mais n'est pas détruite et le domaine chevauchant est scellé de manière étanche par une jonction de soudure à assemblage de matière.
